# EUROPEAN PATENT APPLICATION

(11) **EP 1 130 009 A1**
(43) Date of publication of application: **05.09.2001**
(21) Application number: 00104236.5
(22) Date of filing: 01.03.2000
(51) Int. Cl.: C07B 61/00, C07H 15/203, G01N 33/53

(54) **Generation and screening of a dynamic combinatorial library**

(71) Applicant: Therascope AG, 69123 Heidelberg (DE)
(72) Inventor: Lehn, Jean-Marie, Prof, Dr c/o Institut Le Bel, 67000 Strasbourg (FR); Ramström, Olof, Dr c/o Institut Le Bel, 67000 Stasbourg (FR)
(74) Representative: Isenbruck, Günter, Dr.

(57) **Abstract**

The present invention concerns a method for selectively establishing a dynamic combinatorial library of ligands binding to a target which binds at least two functionalities, which method comprises the following steps:
- selecting a plurality of functionalities which upon combination with each other are capable of forming an antity which may bind to the at least two functionalities in the target;
- linking at least two identical or different functionalities by at least one spacer group allowing reversible bond formation, thus creating discrete ligands;
- mixing together a plurality of a different discrete ligands having different combinations of functionalities;
- subjecting the mixture to conditions allowing a reversible bond formation and cleavage, hence a scrambling of the formalities;
- analyzing the mixture obtained;
- adding the target to the mixture;
- again analyzing the mixture, comparing the results obtained and identifying the functionality combinations which are most appropriate for the formation of a bond.

In a further embodiment of the invention, the target is added when the discrete ligands are mixed together, in order to be present when the scrambling takes place.

## Description

### Technical Field and Prior Art

The present invention relates to a method for selectively producing a dynamic combinatorial library (DCL) for binding to a target. More specifically, the dynamic combinatorial library which is established with the method according to the present invention is directed to molecules which contain at least two functionalities which are capable of binding to the target and which functionalities are linked by a dynamic spacer group allowing an interchange between the functional groups.

New chemical or biological entities with useful properties are generated by identifying a chemical or biological compound (a so-called lead compound) with some desirable properties or activities, creating varieties of said compound to form a library, and evaluating the properties and activities of those variant compounds.

The drawback of that method is that only a small sub-set of the library is usually accessible for biological tests.

Thus, the conventional approach is limited by the relatively small pool of previously identified compounds which may be screened to identify new compounds with the desirable property or activity.

Another drawback pertains to the step of the creation of variants. Traditionally, compound variants are generated by chemists or biologists using a conventional chemical or biological synthesis procedure. Thus, the generation of compound variants is time-consuming and requires huge amounts of work.

Recently, attention has focused on the use of combinatorial chemical libraries to assist in the generation of new chemical compounds.

Combinatorial chemistry has experienced an explosive growth in recent years, as it provides a powerful methology for exploring the molecular geometrical and interactional spaces through molecular diversity generation. This is in particular the case for the discovery of new biologically active substances and medical drugs. It resides on the constitution of vast combinatorial libraries (CLs), extensive collections of molecules derived from a set of units connected by successive and repetetive application of specific chemical reactions. It is thus based on a large population of different molecules that are present as discrete entities.

In the spirit of Emil Fischer's "Lock and Key" metaphor the constitution of a CL of substrates amounts to the fabrication of a large collection of keys, with the goal that one of them will fit the target lock/receptor and be retrievable from the mixture.

In contrast to this, the present invention makes use of a different concept to establish combinatorial libraries, namely of the so-called dynamic combinatorial chemistry which is a conceptionally different approach that rests on supramolecular chemistry. It relies on a reversible connection process for the spontaneous and continuous generation of all possible combinations of a set of basic components, thus making virtually available all structural and interactional features that these combinations may present. Such multicomponent self-assembly amounts to the presentation of a dynamic combinatorial library (DCL) which is a potential library made up of all possible combinations in number and nature of the available components. This is followed by selection of the component among all those possible, that possesses the features most suitable for formation of the optimal supramolecular entity with the target site, by recruiting the correct partners from the set of those available. The degree of completeness of the set of components/subunits depends on the extent to which the possible combinations cover the geometrical and interactional spaces of the targets.

Self-assembly in a multi-component system is a combinatorial process with a search procedure directed by the kinetic and thermodynamic parameters imposed by the nature of components and their interactions.

In WO 97/43232 there is disclosed a substance library which library consists of molecular species which are bonded to a molecular pairing system. The pairing system is composed of molecules, in the preferred embodiment, which are selected amongst specially designed nucleic acids which can bind to each other in a certain manner which results in a particular geometric form. The molecular species are selected, in a preferred embodiment, from the group consisting of peptides, and these peptides are designed according to the particular requirements of a given substrate which is brought into contact with the library component. The complex which forms upon contact with the substrate is identified, in order to evaluate the interaction between a substrate and complex.

In Proc. Natl. Acad. Sci. USA 1997 (94), 2106, Ivan Huc and Jean-Marie Lehn disclose a method for the generation of a dynamic combinatorial library of imines from structural fragments bearing aldehyde and amino groups. The method is directed toward the synthesis of inhibitors of the enzyme carbonic anhydrase by recognition-involved assembly, and the synthesis of the above-mentioned imines is carried out in the presence of the said enzyme carbonic anhydrase. It was found that reversible combination of the used amines and aldehydes leads to the shift of the equilibrium population towards the imine product that was closest in structure to a known highly efficient inhibitor of the enzyme.

### Detailed Description of the Invention

The problem underlying the present invention is to find a method for creating a dynamic combinatorial library which library is composed of molecules which contain at least two discrete functionalities capable of binding to the target and which method allows an interchange of the functionalities in order to create combinations of different functionalities which interact with the receptor in different ways.

This object is attained by a method for selectively establishing a dynamic combinatorial library of ligands for a target which binds at least two functionalities, which method comprises the following steps:
(i) selecting a plurality of functionalities which upon combination with each other are capable of forming an entity which may bind to the at least two functionalities in the target;
(ii) linking at least two identical or different functionalities by at least one spacer group allowing reversible bond formation, thus creating discrete ligands;
(iii) mixing together a plurality of a different discrete ligands having different combinations of functionalities;
(iv) subjecting the mixture to conditions allowing a reversible bond formation and cleavage, hence a scrambling of the functionalities;
(v) adding the target to the mixture;
(vi) identifying the functionality combinations which are most appropriate for the formation of a complex between the target and the active molecule.

This object is also attained by a method for selectively establishing a dynamic combinatorial library of ligands for a target which binds at least two functionalities, which method comprises the following steps:
(I) selecting a plurality of functionalities which upon combination with each other are capable of forming an entity which may bind to the at least two functionalities in the target;
(II) linking at least two identical or different functionalities by at least one spacer group allowing reversible bond formation, thus creating discrete ligands,
(III) mixing together a plurality of different discrete ligands having different combinations of functionalities in the presence of the target;
(IV) subjecting the mixture to conditions allowing a reversible bond formation and cleavage, hence a scrambling of the functionalities;
(V) identifying the functionality combinations which are most appropriate for the formation of a complex between the target and the active molecule.

In one embodiment of the methods described above, the final mixture is analysed and the result compared to the result obtained on a mixture obtained under identical conditions, but without the addition of the target.

It was found that it is possible to generate dynamic combinatorial libraries (DCL's) by linking functionalities with a dynamic spacer group in which reversible covalent bonds can easily and reversibly be formed and broken, hence allowing for an interchange reaction along the said spacer group.

The method may thus be useful for determining which part of the molecule is responsible for the affinity of the target.

Thus, the compounds that are assayed are fragmented in basic components by splitting a reversible bond between two parts of the molecule under conditions allowing such a splitting.

By allowing the basic components to reassemble and compete for the target, it is thus possible to determine which compound or moiety has an affinity for the target and eventually which compound or moiety has the best affinity for the target.

The concept of the invention is based on an approach that rests on supramolecular chemistry.

The method according to the present invention is in particular appropriate for the generation of libraries of molecules for target substrates in which one active binding site is specific for two or more functionalities. This is a preferred embodiment of the present invention. However, the method is also appropriate for the generation of libraries of molecules which bind to two or more different binding sites in a given target

When in the context of the present invention there is referred to "functionality", this includes, on the one hand, simple chemical functional groups like, for example, amino groups and derivatives thereof, amido groups, hydroxyl groups, carbonyl groups, carboxylate and ester groups, sulfone groups, sulfonamide groups, thiol groups, thiocarboxylate and thioester groups.

On the other hand, this term also includes higher entities which themselves may contain functional groups, and non-limiting examples include heterocycles carrying one or more heteroatoms in the ring selected from the group consisting of N, O and S, amino acids and oligo- and polypeptides, sugars and sugar derivatives, nucleic acid constituents and related groups-

According to the invention, the terms used therein have the following meanings:
"ligand" means a chemical or biological molecule with a molecular weight typically not greater than 1000, preferably not greater than 500, advantageously not greater than 200, which possesses an affinity for a target, i.e. that is able to interact with the target by forming simultaneaously a plurality of weak bonds such as hydrogen bonds, hydrophobic interactions etc.
"target" means a biological macromolecule with a molecular weight typically greater than 500, preferably greater than 1000, which is of proteinaceous nature, including lipoproteins, glycoproteins and analogs of proteins, wherein either the peptide bond CO-NH- is replaced by an analogous bond, eventually reversible such as an imine, ester, sulfonamide, sulfone, sulfoxide, phosphate, phosphonate, phosphonamide, guanidine, urea, thiourea, or imide bond, or wherein the aminoacids found in natura proteins
"reversible bond" means that the half time of the bond formation or cleavage is typically less than one month, preferably less than one day and more, preferably less than one hour.

An important point in the present invention is the spacer group located between the functionalities which must comprise in its chain atoms or functional groups which allow a reversible formation and cleavage of bonds and hence a scrambling process resulting in an interchange of functionalities. Prerequisites an appropriate spacer group has to fulfill are, besides the reversibility of the bond formation, an easy controllability, the possibility to be operated under mild conditions and the compatibility with the binding of the functionality to the target.

In one particular embodiment of the present invention, the spacer group contains a disulphide bond. The disulphide bond has several advantages when used in a dynamic combinatorial protocol. Firstly, disulphides undergo rapid interchange with thiols at moderate to high pH-values (≥ 7), but the bond formed is stable at a low pH (< 5), thus allowing to lock/unlock the dynamic process by a simple change in pH. Secondly, the reactivity of non-aromatic disulphides is reasonibly similar, and the equilibrium constant is close to unity, e.g. to isoenergetic behaviour. Thirdly, the interchange can be performed in aqueous environment under mild conditions. Finally, the reaction is highly chemoselective so that only thiol-disulphide interchange occurs in the presence of other chemical functionalities.

The present invention is however not limited to the use of disulphide bridges as a scrambling-permitting entity in the spacer group. Other reversible bond types that can be used to this end in the present invention are, for example, imines and their analogues, acetals, esters and also alkenes wherein scrambling is rendered possible by a metathesis reaction.

Preferably, the spacer group allows to lock the process by a simple reaction. When designing a dynamic library making use of the method to the present invention, two different procedures can be employed. In one procedure, library generating and screeening is performed in one single step; in the other procedure, these two steps are carried out separately. The particulars for the above-mentioned different procedures are the following:
a) adaptive, combinatorial libraries (one-step procedure): the generation of the library constituents is conducted in a single step in the presence of the target, so that the library composition may adjust leading to selection and amplification of the preferred substrate(s); the DCL may be real or virtual; screening by the target occurs in parallel with the reversible generation of the library constituents; this is the approach where the dynamic features are operative over the whole process;
b) pre-equilibrated dynamic combinatorial libraries (pDCL) (two-step procedure): the constituents of the library are generated by reversible interconversion and equilibration in absence of the target, which is added in a second step after reversibility has been stopped; this has the advantage that one may use reversible reactions which are not compatible with the presence of the target but the process is not adaptive and no amplification of the preferred substrate can result; it is however sufficient for lead generation, i.e. the discovery of species presenting the activity sought for; in its second phase it amounts to the usual, static combinatorial chemistry approach where an actual, real library is screened by the target; the resulting library may be termed pre-equilibrated or postdynamic combinatorial library (pDCL).

Generally, the target is a natural protein, namely an enzyme, a receptor or an antibody. Receptors may be membrane receptors, hormone receptors, signal transducers, etc.

When the target is an enzyme, the ligand that is sought to be obtained may act as a substrate, an inhibitor or an activator for said enzyme.

When the target is a receptor, the partner that is sought to be obtained may act as a natural or artificial ligand, an agonist or an antagonist for said receptor.

When the target is an antibody, the partner that is sought to obtain will act as an antigen for said antibody.

The basic components are selected according to their chemical structure.

In a more preferred embodiment, the method according to the present invention is used to design libraries of molecules in which the two or more functionalities are selected from the group consisting of sugars, for example pentoses and hexoses which may be chemically modified, if desired. This is important in so far as carbohydrate recognition plays an important role in many biological processes, such as cell-cell interactions and cell communication. In addition, a multitude of enzymes are involved in various carbohydrate mediated processes associated with, e.g., defence, cell proliferation and cell death, as well as in general carbohydrate metabolism. Carbohydrate groups are therefore highly attractive tools for generating mimics and analogues of such recognition processes. Possible applications are, for example, the direct or indirect inhibition of carbohydraterecognizing enzymes or the finding of potential agonists/antagonists of carbohydrates receptors. The method according to the present invention allows to design libraries of compounds which are capable of being applied in those fields.

In the most preferred embodiment of the present invention, the process is used to establish a DCL for the binding of carbohydrates to those proteins belonging to the class of lectins. One example for lactins is Concanavalin A (Con A) which is specific for a branched trimannoside core unit, located in N-glycosidic carbohydrate-peptide linkages of glycoproteins often associated to cell surfaces, which is the reason why Con A is extensively used as a tool in histochemical staining.

The binding site of Con A is rather shallow, composed of a number of threonine and tyrosine hydroxyl, aspartate carboxyl, as well as asparagine and main chain amide groups, capable of forming a network of hydrogen bonds to the natural substrate, the branched trimannoside unit (Man)₃. The majority of these interactions are formed with the non-reducing, peripheral mannosides of (Man)₃, rather than with the central mannose group, thus implying that the latter acts more or less as a linker between the interacting parts.

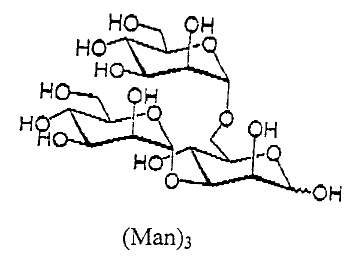

It was possible to mimic this interaction by a structure containing two carbohydrate head groups joined by a spacer containing a reversible covalent bond as will be described in the following.

### Modes of carrying out the Invention

The design of the initial carbohydrate library was based on considerations concerning the mimicking of the native trimannoside unit. Originally, a size of the linker as close as possible to the situation in trimannoside was aimed for. However, since the Con A binding site is rather shallow and freely accessible to the surrounding pool of solvent, it could be envisaged that a slightly longer, but sufficiently flexible chain, could be used as well. Such a compound would then be able to fold into a conformation suitable for fitting into the site. Obviously, an excessively extended linker would result in a lower binding, because of a higher entropical loss upon binding. Finally, the compounds 1-6 listed in Table 1 were chosen as e first approach to a library of carbohydrate homodimers, containing a disulphide bridge in the linker region. A phenylamino group was also introduced, primarily because it provided a chromophore for the subsequent HPLC-analysis. Three hexopyranosides (D-mannose, D-glucose, D-galactose), and two pentopyranosides (L-arabinose, D-xylose) were used as carbohydrate head groups, and two different linkers, varying in length by one methylene group were examined.

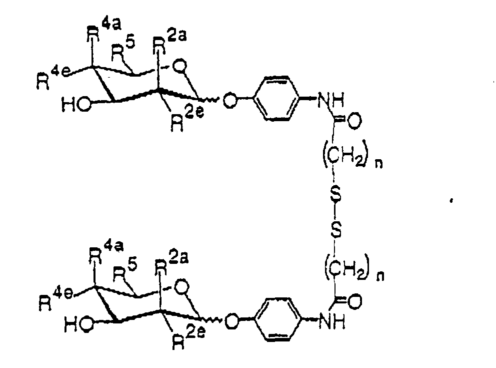

**Table 1**

| Structures of the disulphide linked carbohydrate dimers 1-6 | | | | | | | |
|---|---|---|---|---|---|---|---|
| Compound | α/β | R^{2a} | R^{2e} | R^{4a} | R^{4e} | R⁵, | n |
| 1 (D-Man) | α | OH | H | H | OH | CH₂OH | 3 |
| 2 (D-Gal) | β | H | OH | OH | H | CH₂OH | 2 |
| 3 (D-Gal) | β | H | OH | OH | H | CH₂OH | 3 |
| 4 (D-Glc) | β | H | OH | H | OH | CH₂OH | 2 |
| 5 (L-Ara) | β | H | OH | OH | H | H | 2 |
| 6 (D-Xyl) | β | H | OH | H | OH | H | 2 |

The libraries resulting from scrambling of the original dimers were generated by mixing the dimers 1-6 together with an initiating reagent, capable of reducing some of the disulphides to the corresponding thiols. Dithiothreitol (DTT) was found to be a good choice, since this compound oxidises efficiently to a stable 6-membered cyclic disulphide that may be expected not to take part in the scrambling of the library disulphides. Upon initiation/reduction, interconversion between the disulphides occurred, the rate of which was highly dependent upon the pH of the solution. At high pH (>8), scrambling was achieved reasonably rapidly (within hours), whereas at low pH (<5) no scrambling could be detected. On the other hand, without initiation, no significant scrambling occurred within two weeks. A similar pH-dependence was recorded for the binding of the carbohydrates to the receptor, where a pH close to neutral was preferable. A pH of 7.4 was chosen as a level at which a reasonable rate of scrambling could be obtained, while receptor binding was not significantly affected.

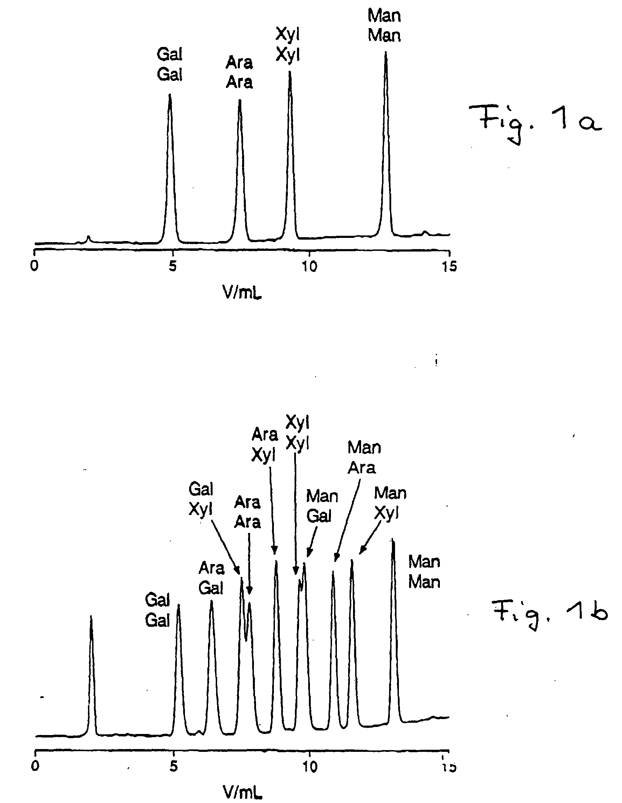

In the case of the library of size 10 (n = 4), in the absence of any receptor, scrambling occurred smoothly at pH = 7.4 from the original species (Figure la) generating all ten expected ditopic combinations in comparable amounts as analyzed by RB-HPLC (Figure 1b). When the receptor in the form of sepharose-bound Con A was added to the equilibrating pool, a shift in the concentrations of the different constituents present unbound in solution was recorded (Figure 1c) The fraction of some of the free species decreased, notably the D-mannose-containing homo- and heterodimers. In order to characterize the species actually bound to the receptor, the Con A-sepharose beads were eluted by acidifying the solution to pH 4 and the composition of the eluate was analyzed (Figure 1d). The assignment of the peaks to the different constituents of the scrambled libraries was made on the basis of the relative retention times. Clearly, the D-mannose homodimer was most efficiently bound to the lectin, and to a lesser extent the D-mannose containing heterodimers. All other species in the equilibrating pool did not bind to Con A-sepharose and remained in solution. Thus, the receptor could be used to "fish out" the best bound species from the equilibrating pool, while leaving the others behind.

When the receptor was present during the entire scrambling process, a slightly higher yield of the bis-mannoside could be recovered, compared to when it was added upon pre-equilibration. In the former case, the amount of bis-mannoside was approximately 2.1 times higher than each of the D-mannose containing heterodimers. In the latter situation, this ratio decreased to 1.5 times. This result is indicative of an adaptive effect where the lectin to some extent acts as a thermodynamic trap during scrambling.

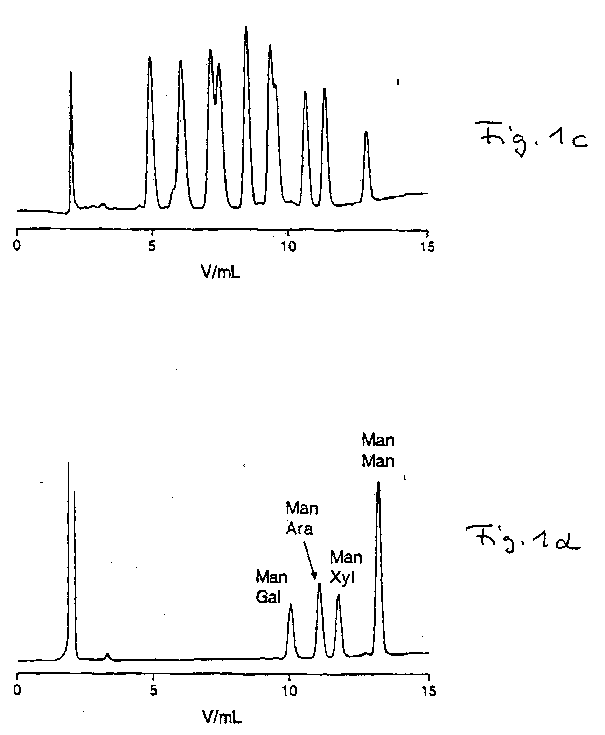

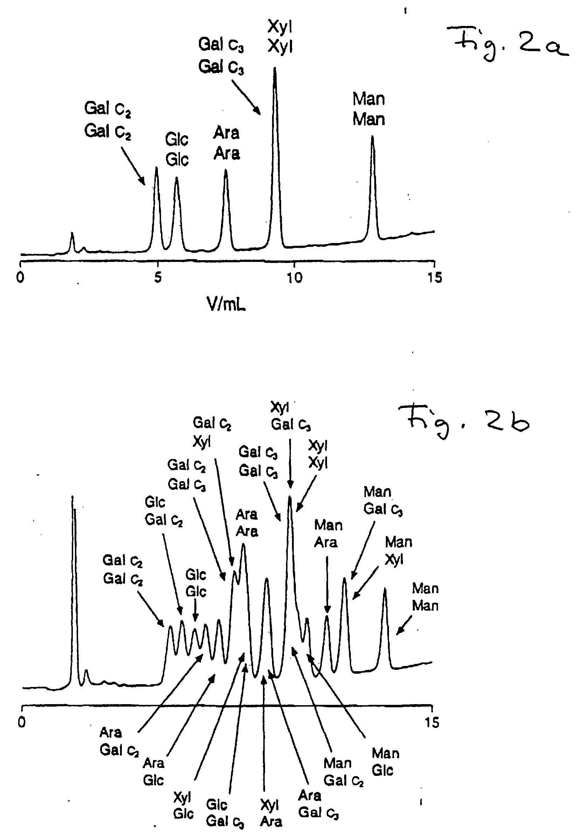

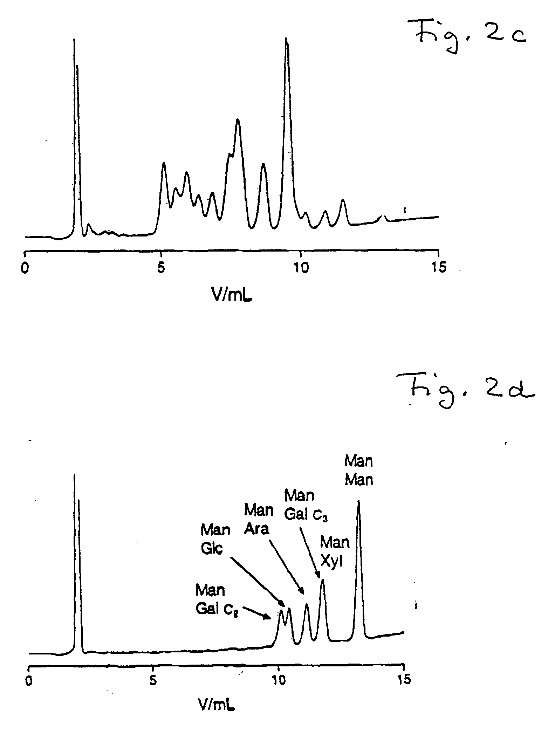

The same behavior, albeit less pronounced was observed for the 21-library (n = 6, Figure 2). Due to the larger number of species resulting from the scrambling, difficulties in their separation were encountered with the system used, and not all components could be resolved in the HPLC conditions used (Figure 2a, 2b). This was particularly the case for the homodimer containing D-galactose and a linker with 3 methylene units (Gal C3), and the D-xylose homodimer (Xyl), which could not be distinguished by reverse phase chromatography (Figure 2a). However, from cross-reference studies the resulting chromatographic separation pattern was, as could be expected, similar to the resulting 10-library. Upon elution of the Con A-sepharose and analysis of the eluate (Figure 2d), a much clearer picture could be attained: mainly the D-mannose homodimer and to some extent the D-mannose containing heterodimers were found to be bound, while all other library constituents remained in the eluate.

Synthesis of the library components: The carbohydrate dimers were synthesized from the corresponding peracetylated 4-aminophenyl-glycosides, by condensation with the bis-dithiodiacids, followed by deacetylation under standard Zemplén conditions (NaOMe/MeOH). The 4-aminophenyl derivatives were all obtained from the commercial 4-nitrophenyl-glycosides following the same procedure.

### Examples

**Synthesis of the library components:** The carbohydrate dimers were synthesised from the corresponding peracetylated 4-aminophenyl-glycosides, by condensation with the bis-dithiodiacids, followed by decetylation under standard Zemplén conditions (NaOMe/MeOH). The 4-aminophenyl derivates were all obtained from the commercial 4-nitrophenyl-glycosides following the same procedure.

**4-aminophenyl-α-L-2,3,4-tri-O-acetyl-arabinopyranoside:** The glycoside (500 mg) was slurried in dichloromethane (2.5 mL) and acetic anhydride (2.5mL). A catalytic amount of dimethylaminophridine (DMAP) was added and the reaction allowed to proceed under argon at ambient temperature overnight. Following washing (water, HCl(aq.), NaHCO₃ (aq.), water), the product solution was concentrated and dried in vacuo. The product was subsequently dissolved in methanol (40 mL), palladium and charcoal was added (5%, 140 mg), and reduction was performed under H₂ for 4 hrs. After filtration of the catalyst, concentration and drying, the pure product was obtained as a white foam (93%). [α]_{D}²⁰ = + 29°(c = 1,0 CWCl₃)¹³C NMR (50 MHz, CDCl₃, 298 K): δ =170.4, 170.2, 169.5, 118.8, 116.0, 100.6, 70.1, 69.2, 67.5, 63.1, 21.0, 20.8; FAB-MS⁺, 367.3 (M⁺).

**4-aminophenyl-β-D-2,3,4-tri-O-acetyl-xylopyranoside:** (94%) [α] _{D}²⁰ = -27° ¹³C NMR (50 MHz, CD₃OD, 25° = 171.7, 151.2, 144.5, 119.6, 117.7, 101.7, 73.3, 72.6, 70.4, 63.2, 20.8; 367.1 (M⁺).

**4-aminophenyl-β-D-2,3,4,6-tetra-O-acetyl-galactopyranoside:** (quant.) [α]_{D}²⁰ = +4.6° (c = 10.0 CHCl₃); ¹³C NMR (50 MHz, CDCl₃, 25°C): δ = 170.4, 170.2, 169.5, 150.0, 142.7, 119.0, 115.9, 101.2, 76.4, 71.0, 68.9, 67.1, 61.4, 20.7, 439.3 (M⁺); (S. Tahutake et al., Chem. Pharm. Bull. 1990(38), 13).

The other 4-aminophenyl derivatives had properties in agreement with the data reported in literature; 4-aminophenyl-β-D-2,3,4,6-tetra-O-acetyl-glucopyranoside, 4-aminophenyl-α-D-2,3,4,6-tetra-O-acetyl-mannopyranoside (D. Pagé et al., Bioorg. Med. Chem. 1996(4), 1949).

The carbohydrate dimers were obtained by the following procedure: A suspension of 4-aminophenyl-glycoside (0.57 mmol), 1-ethyl-3-(dimethylaminopropyl)-carbodiimide (0.63 mmol), and bis-dithiodiacid (0,29 mmol) was stirred for 4 hrs at room temperature under argon in dichloromethane ( 5 mL). Following washing (water, NaHCO₃ (aq.), water), and concentration of the organic phase, the product was purified by chromatography. Subsequent deprotection yielded the pure carbohydrate dimer.

**Bis-mannoside 1:** The intermediate product was purified by flash chromatography (SiO₂; dichloromethane/acetone 8:2, v/v), (67%), [α]_{D}²⁰ = +94° (c = 0.5, MeOH/H₂O 1:1, v/v); ¹³C NMR (50 MHz, CD₃OD, 25°); δ = 172.0, 153.0, 133.0, 121.4, 116.7, 99.1, 73.9, 71.0, 70.6, 67.0, 61.3, 37.5, 34.7, 24.8, ES-MS⁺, 745.5 (M+H).

**Bis-galactoside 2:** Purification by preparative TLC (Chromatotron, Al₂O₃, dichloromethane, 1% MeOH, v/v), (86%), [α]_{D}²⁰ = -46° (c = 0.2 in MeOH/H₂O 1:1, v/v); ¹³C NMR (50 MHz, CD₂OD/D₂O 1:1, 25°C): δ = 171.7, 154.3, 132.4, 122.5, 117.0, 101.5, 75.4, 72.9, 70.7, 68.6, 60.7, 35.7, 33.6; ES-MS⁺ [calc. for C₃₀H₄₀N₂O₁₄S₂ 716.2 found] 739.3 (M+Na); [calc. C 50.27. H 5.62, N 3.91: found C 50.03, H 5.66, N 3,76].

**Bis-galactoside 3:** Purification by preparative TLC (Chromatotron, Al₂O₃, dichloromethane, 0.5% MeOH, v/v) (36%), [α]_{D}²⁰ = -43° (c = 0.08 in MeOH/H₂O 1:1, v/v; (t, ¹³C NMR (50 MHz, CD₃OD/D₂O 1:1, 25°C); δ = 173.4, 154.2, 132.5, 122.6, 117.0, 101.5, 75.4, 72.9, 70.7, 68.6, 60.8, 37.4, 34.8, 24.8; ES-MS⁺ 767.2 (M+Na);

**Bis-glucoside 4:** Purification by flash chromatography (SiO₂; dichloromethane/acetone 8:2, v/v, (67%), [α]_{D}²⁰ = -40° (c = 0.3, MeOH/H₂O 1:1, v/v);¹³C NMR (50 MHz, CD₃OD/D₂O 4:1, v/v, 25°CC): δ = 171.1, 154.2, 132.8, 121.8, 116.9, 101.1, 76.5, 76.2, 73.4, 69.8, 61.0, 35.8, 33.7;

**Bis-arabinoside 5:** Purification by flash chromatography (SiO₂; dichloromethane/acetone 8:2, v/v), (42%), [α]_{D}²⁰ = -6° (c = 0.2, MeOH/H₂O 1:1, v/v); ¹³C NMR (50 MHz, CD₃OD/D₂O 1:1, 25°C): δ = 171.8, 154.1, 132.2, 122.6, 117.1, 101.5, 72.4, 70.6, 68.2, 66.2, 35.7, 33.6, ES-MS⁺ 657.0 (M+H).

Bis-xyloside 6: Purification by preparative TLC, (Chromatotron, Al₂O₃, dichloromethane/1% MeOH, v/v), (63%), [α]_{D} = -21° (c = 0.3, MeOH/H₂O 1:1, v/v); ¹³NMR (50 MHz, CD₃OD/D₂O 1:1, 25°C): δ = 171.8, 153.9, 132.5, 122.6, 117.0, 101.4, 75.8, 73.1, 69.3, 65.3, 35.7, 33.6, ES-MS⁺ 679.2 (M+Na).

**Generation and analysis of adaptive combinatorial libraries:** To a solution of carbohydrate dimers 1-6 (10µM, each) in buffer (900 µL, 100 mM Na-phosphate, 50 µM MnCl₂, pH 7.4), was added suspension of Con A-sepharose (50 µL, -5 nmol Con A), and dithiothreitol solution (50 µLm, 20 mM). After equilibration at ambient temperature for two weeks, the suspension was filtered (Millipore, Amicon, YM-100), and the sepharose particles resuspended in buffer (400 µL). The bound carbohydrate dimers were released by addition of HCl (100 µL, 1 M), and thereafter renewed filtration, the solutions were analysed by RP-HPLC.

**Generation and analysis of pre-equilibrated combinatorial libraries:** The same protocol as for the adaptive libraries was used with the exception that Con A-Sepharose (50 µL) was added after the equilibration period. The resulting suspension was then allowed to further equilibrate during 1 hr at room temperature, and the bound species were eluted and analysed as previously mentioned.

## Claims

1. A method for selectively establishing a dynamic combinatorial library of ligands for a target which binds at least two functionalities, which method comprises the following steps:
(i) selecting a plurality of functionalities which upon combination with each other are capable of forming an entity which may bind to the at least two functionalities in the target;
(ii) linking at least two identical or different functionalities by at least one spacer group allowing reversible bond formation, thus creating discrete ligands;
(iii) mixing together a plurality of a different discrete ligands having different combinations of functionalities;
(iv) subjecting the mixture to conditions allowing a reversible bond formation and cleavage, hence a scrambling of the functionalities;
(v) adding the target to the mixture;
(vi) identifying the functionality combinations which are most appropriate for the formation of a complex between the active target and the active molecule.

2. A method for selectively establishing a dynamic combinatorial library of ligands for a target which binds at least two functionalities, which method comprises the following steps:
(I) selecting a plurality of functionalities which upon combination with each other are capable of forming an entity which may bind to the at least two functionalities in the target;
(II) linking at least two identical or different functionalities by at least one spacer group allowing reversible bond formation, thus creating discrete ligands,
(III) mixing together a plurality of different discrete ligands having different combinations of functionalities in the presence of the target;
(IV) subjecting the mixture to conditions allowing a reversible bond formation and cleavage, hence a scrambling of the formalities;
(IV) identifying the functionality combinations which are most appropriate for the formation of a complex between the target and the active molecule.

3. The method according to claim 1 or 2. wherein the final mixture is analysed and the result compared to the result obtained on a mixture obtained under identical conditions in the absence of the target.

4. The method according to any of the claims 1 to 3, wherein the said two or more functionalities are bound in one site in the target.

5. The method according to any of the claims 1 to 4, wherein the functionality is selected from the group consisting of heterocycles containing at least one atom selected from the group consisting of N, O and S, amino acids, oligo- and polypeptides and sugars and its derivatives and nucleic acid constituents and related groups.

6. The method according to any of the claims 1 to 5, wherein the functionality is a sugar, preferably a sugar selected from the group consisting of hexoses and pentoses.

7. The method according to any of the claims 1 to 6, wherein the spacer group is selected from the group consisting of amines, acetals, esters, alkenes and disulphides, preferably disulphides.

8. The method according to claim 7, wherein the reversible bond cleavage and formation is attained by the addition of dithiotreitol.

9. The method according to any of the claims 1 to 8, wherein the target is a protein, an enzyme, a biological receptor or an antibody.

10. The method according to claim 9, wherein the said enzyme is a carbohydrate binding protein, preferably a lectin.

11. The method according to claim 9, wherein said ligand is selected from the group consisting of a substrate, an activator and an inhibitor of an enzyme, the target being an enzyme or an analogue thereof.

12. The method according to claim 9, wherein said ligand is selected from the group consisting of ligands, agonists and antagonists of a receptor.

13. The method according to claim 9, wherein said ligand is an antigen, said target being an antibody.

14. A dynamic combinatorial library which is obtainable by the method according to any of claims 1 to 13.
